# EUROPEAN PATENT SPECIFICATION

(11) **EP 4 178 536 B1**
(45) Date of publication and mention of the grant of the patent: **29.10.2025**
(21) Application number: 21751893.5
(22) Date of filing: 06.07.2021
(51) Int. Cl.: A61K 9/00

(54) **METHODS TO CONTROL THE RATE OF RELEASE OF THERAPEUTIC AGENTS FROM IMPLANTABLE DEVICES**
VERFAHREN ZUR STEUERUNG DER FREISETZUNGSRATE VON THERAPEUTISCHEN MITTELN AUS IMPLANTIERBAREN VORRICHTUNGEN
PROCÉDÉS POUR RÉGULER LA VITESSE DE LIBÉRATION D'AGENTS THÉRAPEUTIQUES À PARTIR DE DISPOSITIFS IMPLANTABLES

(30) Priority: 08.07.2020 US 202063049573 P
(43) Date of publication of application: 17.05.2023
(73) Proprietor: Nano Precision Medical, Inc., Emeryville, CA 94608 (US)
(72) Inventor: OLF, Ryan, Emeryville, California 94608 (US); GORDON, Lyle, Emeryville, California 94608 (US); ROORDA, Wouter, Emeryville, California 94608 (US)
(74) Representative: J A Kemp LLP
(86) International application number: PCT/US2021/040444
(87) International publication number: WO 2022/010846

(56) References cited:
- US-A1- 2018 303 759
- US-A1- 2020 040 140

## Description

### CROSS-REFERENCES TO RELATED APPLICATIONS

This application claim priority to US Provisional Patent Application No. 63/049,573, filed July 8, 2020.

### BACKGROUND

Many subjects, humans as well as animals, are in need of long-term treatment with therapeutic agents. In order to improve adherence, many subjects would benefit from the adherence provided by an implantable device releasing a desired therapeutic agent at a desired rate for an extended period of time. US 2020/040140 A1 describes polymeric stabilizing formulations. Despite many years of research, there is still a need for the development of such devices, and specifically for methods to control the rate of release of therapeutic agents from such devices upon implantation in a subject to be treated. The present disclosure satisfies this need and offers additional advantages as well.

### BRIEF SUMMARY

In an aspect, the present disclosure provides use of a pH controlling agent to control a rate of release of a therapeutic agent, which is a peptide or protein, from an implantable device, the device comprising:
a capsule configured for implantation and having a reservoir;
a fluid formulation of the therapeutic agent contained within the reservoir and having a pH; and
a titania nanoporous membrane with a plurality of pores attached to the capsule and providing a diffusion path for release of the therapeutic agent out of the reservoir;
wherein the rate of release of the therapeutic agent through the nanoporous membrane is dependent on the pH of the formulation; and
wherein the pH of the formulation is selected at a level suitable to achieve a desired rate of release of the therapeutic agent.

Other embodiments will become more apparent when read with the detailed description and figures that follow.

### BRIEF DESCRIPTION OF THE DRAWINGS

FIG. 1 represents a device of the disclosure.
FIG. 2 represents the effect of changes to the pH of the external release rate buffer on the release of a therapeutic agent from a device.
FIG. 3 represents the effect of changes to the pH and composition of an internal formulation buffer on the release of a therapeutic agent from a device.
FIG. 4 represents the effect of changes to the pH of an internal formulation buffer on the release of a therapeutic agent from a device.
FIG. 5 represents the effect of a gradual rise of the pH of an internal formulation buffer on the release of a therapeutic agent from a device.
FIG. 6 represents the effect of the orientation of the membrane disk on the release of a therapeutic agent from a device.
FIG. 7 shows a release rate profile of the present disclosure.

### DETAILED DESCRIPTION

The disclosure pertains to the field of long-term treatment of subjects with implantable devices providing a sustained delivery of therapeutic agents at a controlled rate. The disclosure relates to the use of a pH controlling agent to control a rate of release of a therapeutic agent, which is a peptide or protein, from implantable devices. Such devices include formulations and are useful in methods of treatment of a subject.

### Definitions

"Polypeptides" refers to molecules with a backbone chain of 2 or more amino acid residues. Some polypeptides may have additional associated groups, such as metal ions in metalloproteins, small organic molecules such as in heme proteins, or carbohydrate groups such as in glycoproteins. Associated groups may include side chains covalently bound to the backbone (e.g., liraglutide or semaglutide).

"Peptides" and "Proteins" refers to subgroups of polypeptides. In this disclosure the definition of peptides and proteins follows the practice of the United States Food and Drug Administration, the FDA, which defines peptides as polypeptides with up to 40 amino acid residues, and proteins as polypeptides with more than 40 amino acid residues.

Incretin mimetics refers to agents that act like incretin hormones such as glucagon-like peptide-1 (GLP-1). They bind to GLP-1 receptors and stimulate glucose dependent insulin release, therefore acting as antihyperglycemics. Incretin mimetics of the disclosure include, but are not limited to, exenatide, liraglutide, semaglutide, cotadutide, dulaglutide, albiglutide, lixisenatide, sitagliptin, saxagliptin, alogliptin, and linagliptin. In some embodiments of the disclosure more than one incretin mimetic may be present. In some embodiments of the disclosure the incretin mimetic is exenatide.

Exenatide (natural, recombinant and synthetic, also called exendin-4) refers to amino acid sequence His Gly Glu Gly Thr Phe Thr Ser Asp Leu Ser Lys Gln Met Glu Glu Glu Ala Val Arg Leu Phe Ile Glu Trp Leu Lys Asn Gly Gly Pro Ser Ser Gly Ala Pro Pro Pro Ser.

"Formulation of a therapeutic agent" refers to the actual state in which a therapeutic agent is present in a product or in a product fabrication intermediate, and includes the therapeutic agent, plus, optionally, any used additional therapeutic agents, any used formulation excipients and any used formulation solvents.

"Membrane" refers to a permeable structure allowing mass transport of molecules from one side of the structure to the other through the structure.

"Porous membranes" refers to membranes characterized by the presence of a two-phase system, in which membrane matrix material represents one phase, typically a continuous phase, which is permeated by open channels extending from one side of the membrane to the other, and filled with a second phase, often a fluid phase, through which mass transport through the membrane can take place.

"Dense" or "non-porous membranes" refers to membranes without fluid filled pores. In such membranes mass transport may take place by a dissolution-diffusion mechanism, in which therapeutic agents permeate the membrane by dissolving in the membrane material itself, and diffusing through it.

"Nanoporous membrane" and "nanopore membrane" are used interchangeably, and refer to porous membranes in which the pores have a smallest diameter of less than 1000 nanometer.

"Nanotube membrane" refers to a nanoporous membrane, wherein pores are formed by an array of nanotubes.

"Titania nanotube membrane" refers to an array of titania nanotubes where at least a portion of the titania nanotubes are open at both ends and capable of allowing diffusion from one side of the membrane to the other through the titania nanotubes. In certain instances, the array of titania nanotubes is located on a titanium substrate. In certain instances, the titania nanotube membrane has two faces or sides. A first face or side having an array of titania nanotubes and a second face or side of a titanium substrate. In certain aspects, the array of titania nanotubes are grown on the titanium substrate by electrochemical anodization.

"Molecular diameter" of a polymer refers to the diameter of the sphere of gyration of the polymer, which is a physical measure of the size of a molecule, and is defined as two times the mass weighted average distance from the core of a molecule to each mass element in the molecule.

"Stokes diameter" or "hydrodynamic diameter" refers to the dimension of a molecule plus its associated water molecules as it moves through an aqueous solution, and is defined as the radius of an equivalent hard sphere diffusing at the same rate as the molecule under observation.

"Ion exchange resin" refers to a polymer comprising acidic or basic groups, or a combination thereof, made insoluble, for instance by cross-linking, and capable of exchanging anions or cations, or a combination thereof, with a medium surrounding it.

"Fluid" and "fluid form" as used in this disclosure refers to flowable states of matter and includes, but is not limited to gases, solutions, suspensions, emulsions, colloids, dispersions and the like.

"Fluid contact" refers to an entity being in contact with a fluid.

"Neutral pH" refers to a pH between 6.5 and 7.5.

Implantable devices with nanoporous membranes for the release of therapeutic agents have been described previously, e.g. in US Patents Nos. 9814867 and 9770412 and US Patent Pub. No. US20190091140 and PCT/US2021/019559 (published as WO 2021/173770). It has now been found that the release rate of the therapeutic agents from these devices can be powerfully controlled by the pH of the formulations of the therapeutic agent.

Some embodiments of the disclosure include a device with a cylindrical capsule encapsulating a reservoir, a titania nanoporous membrane affixed to one end of the capsule, and a formulation of a therapeutic agent contained within the reservoir. Release of the therapeutic agent from the reservoir after implantation of the device in a subject is controlled by the nanoporous membrane.

The pH of the formulation of the therapeutic agent is utilized as a further means to control the release rate. Additionally, some embodiments control the duration of release of the therapeutic agent using the orientation of the membrane with respect to the reservoir.

### Devices

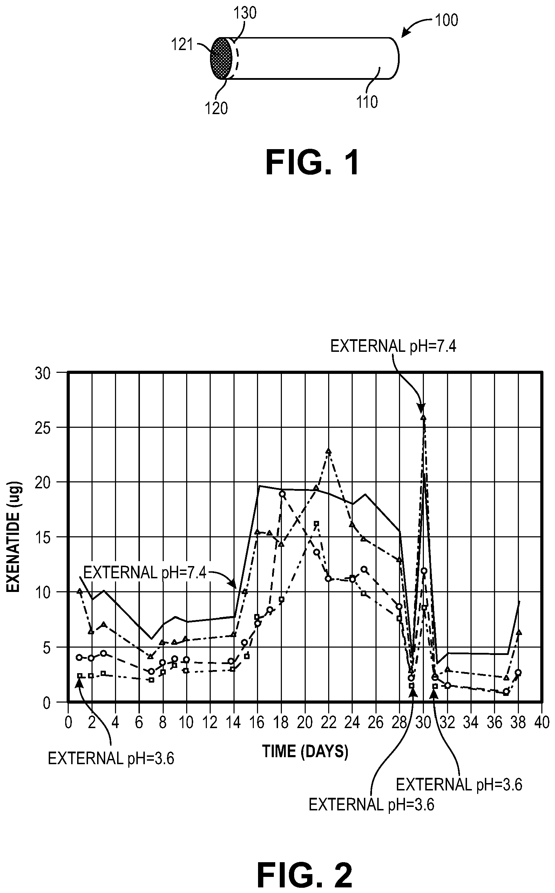

As illustrated in FIG. 1, devices of the disclosure include a capsule 100 suitable for implantation, wherein the capsule has a reservoir 110 suitable for holding a therapeutic agent and a pH controlling agent. In some embodiments, more than one reservoir is present. The capsule may be made of any suitable biocompatible material. In some embodiments, the capsule is made of a medical grade metal, such as titanium or stainless steel, or of a medical grade polymeric material, such as silicone, polyurethane, polyacrylate, polyolefin, polyester, polyamide and the like. In some embodiments, the capsule is made of multiple materials. In some embodiments of the disclosure, the capsule is made of titanium.

Devices of the disclosure have at least one titania nanoporous membrane (120), as described herein, attached to the capsule and in fluid contact with the reservoir, wherein the membrane provides a pathway for mass transport of a therapeutic agent included within the reservoir out of that reservoir and into the body of a subject into which the capsule has been implanted. In this disclosure "attached to the capsule" refers to a component being fixed in place with respect to the capsule, and connected to the capsule directly or indirectly, by using any suitable means, including by welding, gluing, press-fitting and by using threaded means, or by any combination of these. In the case of membranes as described in US Patent 9814867, and as illustrated in FIG. 1, the nanotube membranes are part of an array of nanotubes 121, some of which are still attached to the titanium substrate 130 from which they were grown, and the substrate may be attached to the capsule. At least some of the nanotubes are open on both sides, to allow for mass transport of a therapeutic agent out of the reservoir. In one aspect, the membrane 120 is a titania nanotube membrane 120, which has two faces or sides. A first face or side having an array of titania nanotubes 121 and a second face or side of a titanium substrate 130. FIG. 1 shows the membrane attached to the capsule with the titanium substrate 130 facing towards the reservoir of the device.

Further description of devices of the disclosure may be found in PCT/US2021/019559, published as WO 2021/173770.

### Membranes

A device of the disclosure includes at least one membrane providing a pathway for mass transport of a therapeutic agent out of a reservoir of the device of the disclosure.

The membrane is a titania nanoporous membrane. In some embodiments the membrane is a titania nanotube membrane.

Embodiments of the disclosure are particularly useful as sustained delivery devices for therapeutic agents, in which the release of the agents is controlled by a titania nanoporous membrane.

Fabrication of membranes of the disclosure is described in US Patent 9814867 and control of the internal diameter of the nanopores is described in US Patent 9770412

Further description of membranes of the disclosure may be found in PCT/US2021/019559, published as WO 2021/173770.

### Formulations

Devices of the disclosure include a formulation having at least one therapeutic agent, for instance therapeutic agents such as described in this disclosure. The therapeutic agent may be in solid or fluid form. In some instances, the therapeutic agent may be present in mixed forms, such a suspension of a solid form of the therapeutic agent in a saturated solution of the therapeutic agent. The formulation is in fluid form. Formulations in fluid form, for instance formulations including a solution of at least part of the therapeutic agent in the reservoir, have a pH.

Further description of formulations of the disclosure may be found in PCT/US2021/019559, published as WO 2021/173770.

### pH controlling agents

Materials to control the pH may be the therapeutic agent itself, low molecular weight pH stabilizers, such as acidic and basic compounds, including weakly acidic and weakly basic compounds that can be used as buffering agent, or high molecular weight compounds like poly-acids or poly-bases. Many such compounds are known in the literature, and those with ordinary skills in the art of pharmaceutical formulation development will be able to select suitable ingredients for the formulation without undue experimentation.

In some embodiments the pH controlling materials are insoluble polymeric stabilizers as described in PCT/US2021/019559, published as WO 2021/173770.

Other pH controlling agents suitable for the disclosure can be found in US Patents Nos. 10045943, and 10479868.

"Acid" refers to a compound that is capable of donating a proton (H⁺) under the Bronsted-Lowry definition, or is an electron pair acceptor under the Lewis definition. Acids useful in the present disclosure include Bronsted-Lowry acids that include, but are not limited to, alkanoic acids or carboxylic acids (formic acid, acetic acid, citric acid, lactic acid, oxalic acid, etc.), sulfonic acids and mineral acids, as defined herein. Mineral acids are inorganic acids such as hydrogen halides (hydrofluoric acid, hydrochloric acid, hydrobromice acid, etc.), halogen oxoacids (hypochlorous acid, perchloric acid, etc.), as well as sulfuric acid, nitric acid, phosphoric acid, chromic acid and boric acid. Sulfonic acids include methanesulfonic acid, benzenesulfonic acid, p-toluenesulfonic acid, triflouromethanesulfonic acid, among others.

"Base" refers to a compound capable of accepting a proton (H⁺) under the Bronsted-Lowry definition, or is an electron pair donor under the Lewis definition. Representative bases include, but are not limited to, hydroxy, alkylhydroxy, amines (-NRR), alkylamine, arylamine, amide (-C(O)NRR), sulfonamide (-S(O)₂NRR), phosphonamide (-P(O)(-NRR)₂), carboxylate (-C(O)O-), and others.

In certain instances, the pH adjusting agent is a buffer. The buffer may be selected from the group consisting of citrate/citric acid, acetate/acetic acid, phosphate/phosphoric acid, formate/formic acid, propionate/propionic acid, lactate/lactic acid, carbonate/carbonic acid, ammonium/ammonia, edentate/edetic acid, and combinations thereof.

### Therapeutic agents

Therapeutic agents have been described in PCT/US2021/019559, published as WO 2021/173770. The therapeutic substance is a peptide or protein. In some embodiments the peptide or protein is an incretin mimetic. In some embodiments the incretin mimetic is exenatide. Exenatide is used for the treatment of Type II Diabetes Mellitus, and is under investigation for treatment of other diseases and conditions, such as obesity and Non-Alcoholic Steatohepatitis.

### Manufacture

Methods of manufacture of devices and formulations are described in PCT/US2021/019559, published as WO 2021/173770.

### Methods

Devices of the disclosure have the capability to release therapeutic agents, contained in the reservoir, through the nanopores of the membrane at a controlled rate. In some instances, the rate of release of the therapeutic agent is a non-Fickian release rate, i.e. a release rate that is not proportional to the concentration gradient driving the release. Examples of non-Fickian release rates through nanoporous membranes have been described in US Patent No. 9814867.

The exact mechanism by which the nanopores of the membranes control the release rate is not understood in detail. Interactions between the diffusing molecules of the therapeutic agent and the interior wall of the nanotubes could play a role in this mechanism.

Experimentally, it was found that the rates of release of a therapeutic agent through the membrane may be strongly dependent on the level of the pH of the formulation of the therapeutic agent in the reservoir.

In experimental studies with embodiments of the disclosure, it was found that the release rates of exenatide strongly depended on the pH of the formulation of the therapeutic agent. As can be seen in Examples 1-4 and FIGS. 2-5, a significant increase in release rate was observed over a pH range of about 3.6 to 7.4, with low release rates observed at lower pH levels and high release rates observed at higher pH levels.

The net charge state of many substances is pH dependent. At low pH, in the presence of an abundance of H⁺ or H₃O⁺ ions in an aqueous solution, substances tend to have a net positive charge. At high pH, in the presence of an abundance of OH- ions in an aqueous solution, substances tend to have a net negative charge. The Isoelectric Point (IEP, PI) of a substance is the pH at which the substance has a net neutral charge.

Experimentally, the IEP of exenatide was determined to be 5.46, as measured by capillary isoelectric focusing. (http://en.cnki.com.cn/Article en/CJFDTotal-SWTX201403019.htm, Internal Communications).

The IEP of the interior surface of the nanotubes of membranes of the disclosure is not known with certainty. Generally, literature data indicate a range of pH 3-6, and in-house model studies indicated a pH of about 4.5.

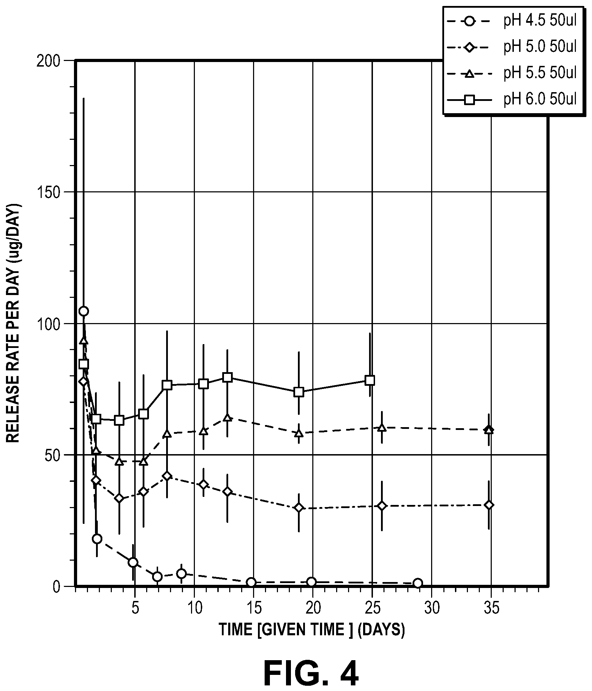

As can be seen in FIG. 4, the release rate of exenatide through membranes of the disclosure is strongly dependent on the pH of the exenatide formulation. At a pH of 4.5, almost no release was observed, and the rates of release gradually increased with increasing pH of the formulation up to pH 6.0.

Without being bound by any particular theory, this may be explained by changing electrostatic interactions between the therapeutic agent such as exenatide and the interior surface of the nanotubes. At pH 4.5, exenatide is present in predominantly positively charged form. Assuming an IEP of 4.5 for the interior surface of the nanotubes, an equal number of positively and negatively charged sites is present. At this pH, almost no release is observed. As the pH gradually rises, both the exenatide and the interior surface of the nanotubes become increasingly negatively charged, and the electrostatic interactions change. At the same time, an increase in release rate is observed. At pH 6.0, both the exenatide and the interior surface of the nanotubes are predominantly negatively charged, and a high rate of release is observed. Therefore, an increasing level of charge similarity between the therapeutic agent and the interior surface of the nanotubes was associated with increasing release rates. In other words, as the negative charge in the interior surfaces and exenatide increase, so too does the release rate.

Many peptides and proteins, including exenatide, have a decreasing stability profile above pH 6.0 as a consequence of the presence of an asparagine residue in the peptide backbone that is sensitive to deamidation reactions above that pH. In certain aspects, pH-dependent windows may exist for the delivery of therapeutic agents through membranes of the disclosure, where pH effects determine release rate, while pH effects may also affect stability of the therapeutic molecule.

In other instances, different considerations may exist for such windows. Other molecules, including peptides and proteins, may have different stability profiles in relation to the pH of their environment. Alternatively, or additionally, pH dependent solubility effects may exist. Within such windows, pH can be a powerful tool to control the release rate of the therapeutic agent through the membrane.

The range of suitable pH levels will depend on the type of nanoporous membranes used, and on the type of therapeutic agents to be delivered. In some instances, therapeutic agents may have substantial stability at pH levels higher than 6.0, and would allow for a much higher upper limit of the pH window of the formulation. In some instances, therapeutic agents may have a substantially higher IEP and may actually require a pH window at a higher pH.

Similarly, nanoporous membranes other than titania membranes may have a different IEP, and require a different pH window.

Suitable pH windows for various combinations of nanoporous membranes and therapeutic agents can be determined by those with ordinary skills in the art of pharmaceutical development, based on literature data and on standard types of release rate experiments.

PCT/US2021/019559, published as WO 2021/173770, discloses the use of pH controlling agents to stabilize therapeutic agents in formulations of the disclosure. In certain instances, the pH controlling agents are insoluble polymeric agents with a plurality of pH sensitive stabilizing groups that can be employed to provide buffering capacity at desirable pH levels, such as weakly acidic or weakly basic groups, to provide chemical stabilization for therapeutic agents in devices of the disclosure. These polymeric agents stabilize the therapeutic agents by controlling the pH of formulations of the disclosure. As disclosed herein, pH controlling agents, such as disclosed in PCT/US2021/019559, published as WO 2021/173770, were now found to be able to control release rates as well.

In some instances, a gradual rise of the release rate of a drug from an implant over time is considered desirable. For instance, with exenatide a gradual ramp-up of the delivered dose per day has been associated with a reduced incidence of nausea. In some embodiments of the disclosure the initial internal pH of a device is set at a relatively low level, and is allowed to rise over time as the internal pH slowly equilibrates with the external environment of the device, i.e. interstitial fluid. The gradual rise in pH is accompanied by a gradual increase in release rate.

In some instances, a dry formulation of a therapeutic agent may be present in a device at the time of implantation in a subject. In such instances a promotor of water uptake may be present in the reservoir, such as a water-soluble gas. After implantation the water-soluble gas may promote the uptake of interstitial fluid into the reservoir through the membrane of the device. Embodiments of the disclosure may include a dry formulation in the reservoir with a composition that, after uptake of the interstitial fluid, generates a liquid formulation with a pH that provides a desired release rate of the therapeutic agent.

Control of rate of release of therapeutic agents from a reservoir through a nanotube membrane can be achieved by controlling the pH of a formulation in the reservoir.

Formulations to control the rate of release of therapeutic agents from a reservoir through a nanotube membrane, wherein the formulation controls the rate of release can be achieved by controlling the pH.

The disclosure provides devices for a controlled rate of release of therapeutic agents, wherein the devices contain the therapeutic agents as well as pH controlling agents.

In some embodiments the pH controlling agents are polymeric stabilizers such as described in PCT/US2021/019559, published as WO 2021/173770. In some embodiments the release rate is controlled by controlling the pH with soluble pH controlling stabilizers, such as low molecular weight acids or bases.

In addition, release rates can be controlled by the number and size of the membranes present on devices of the disclosure. However, for implantable devices, limiting the size of the device typically facilitates the implantation procedure and improves the acceptability of the device from a patient perspective. Therefore, optimizing release rate by optimizing transport rates through a membrane are normally preferred over optimizing the release rate by enlarging the membrane or increasing the number of membranes.

Experimentally, it was found that in some studies an accelerated decline of the release rate occurred at certain time points. The time points were variable between studies, but were generally observed between about 4 and 7 weeks

Membranes of the disclosure are prepared by growing an anodized layer of titanium oxide nanotubes on a titanium substrate (US Patent 9814867). Therefore, such membrane disks have a titanium substrate layer on one side, and a layer of titanium side nanotubes on the other. Experimentally, it was found that by attaching the membrane to the capsule with the titanium substrate layer facing in, i.e. towards the reservoir, the accelerated decline could effectively be prevented. Nanotubes, as prepared according to US Patent 9814867 are somewhat tapered in nature, with the narrow diameter of the taper towards the titanium substrate layer. A configuration with a narrow taper towards the reservoir could be less sensitive to clogging of the nanopores by nanoparticulate matter present on the reservoir side of the membrane.

A number of release rate studies were performed to support these concepts. In these studies, the devices releasing therapeutic agents were placed in an environment mimicking the environment of use of these devices, i.e. the interstitial fluid in the subcutaneous pocket in which the device is implanted. Devices containing formulations at a variety of pH levels and with different pH controlling agents were tested by submerging them in release rate buffers at physiological pH and ionic strength, for instance NaCl/bis-tris buffer at pH 7.4.

The release rates of exenatide were measured by submerging the devices in a controlled amount of release rate medium, changing the release rate medium at selected intervals and measuring the amounts of released exenatide in the release rate medium.

In certain embodiments, the amount of therapeutic agent delivered per day can be from about 0.1 µg to about 1000 µg per day such as 0.1 µg to about 750 µg, or about 1 µg to about 500 µg, or about 1 µg to about 100 µg, or about 1 µg to about 50 µg or about 1 µg to about 15 µg, or about 1 µg to about 10 µg or about 1 µg to about 5 µg per day or about 5 µg to about 500 µg, or about 5 µg to about 100 µg, or about 5 µg to about 50 µg. The amount delivered can be continuous release.

In some instances, larger or smaller amounts per day can be released. In certain embodiments, the therapeutic agent is exenatide. In some embodiments the amount of exenatide delivered per day can be from about 1 µg to about 500 µg per day such as about 1 µg to about 250 µg, or about 1 µg to about 100 µg per day, or about 1 µg to about 50 µg, or about 1 µg to about 25 µg, or about 1 µg to about 15 µg, or about 1 µg to about 10 µg, or about 1 µg to about 5 µg per day, or about 5 µg to about 500 µg, or about 5 µg to about 100 µg, or about 5 µg to about 50 µg per day, or about 15 µg to about 500 µg, or about 15 µg to about 100 µg, or about 15 µg to about 50 µg per day. The amount delivered can be continuous release. In some instances, larger or smaller amounts per day can be released.

In some instances, the volume of reservoir is such that the duration of the amount delivered per day, which can be from about 1 µg to about 500 µg per day, will last for at least 10 days, at least 30 days, at least 45 days, at least 60 days, at least 75 days, at least 90 days, or at least 120 days or even longer.

The doses of exenatide suitable for the treatment of type 2 diabetes can provide any suitable mean steady-state plasma concentration of the therapeutic agent in the subject. For example, the mean steady state plasma concentration can be from 10 pg/ml to 10,000 ng/ml. In some embodiments, the mean steady state plasma concentration for exenatide can be from 50 pg/ml to 600 pg/ml. In some embodiments, the mean steady state plasma concentration for exenatide can be from 170 pg/ml to 350 pg/ml. In some embodiments, the mean steady state plasma concentration for exenatide can be from 170 pg/ml to 290 pg/ml.

In certain embodiments, the amount of exenatide delivered per day is at a pH of about 4.0 to about 7.0, or about 4.1 to about 6.5, or about 4.2 to about 6.5, or about 4.3 to about 6.5or about 4.4 to about 6.5, or about 4.5 to about 6.5, or about 4.6 to about 6.5, or about 4.7 to about 6.5 or about 4.8 to about 6.5, or about 4.9 to about 6.5, or about 5.0 to about 6.5, or about 5.1 to about 6.5, or about 5.2 to about 6.5, or about 5.3 to about 6.5, or about 5.4 to about 6.5, or about 5.5 to about 6.5, or about 5.6 to about 6.05 or about 5.7 to about 6.5, or about 5.8 to about 6.5, or about 5.9 to about 6.5.

In certain embodiments, the amount of exenatide delivered at a pH of about 4.0 to about 6.0 per day or about 5.0 to about 6.0, or about 5.5 to about 6.0, can be from about 1 µg to about 500 µg per day such as about 1 µg to about 250 µg, or about 1 µg to about 100 µg per day, or about 1 µg to about 50 µg, or about 1 µg to about 25 µg, or about 1 µg to about 15 µg, or about 1 µg to about 10 µg or about 1 µg to about 5 µg per day or about 5 µg to about 500 µg, or about 5 µg to about 100 µg, or about 5 µg to about 50 µg per day. The amount delivered can be continuous release. In some instances, larger or smaller amounts per day can be released.

In certain embodiments, the present disclosure provides a seamless transition from a first mode of administration of a therapeutic agent to a second different mode of administration. For example, the present disclosure provides a transition from a subcutaneous administration of exenatide to an implantable device having a reservoir of exenatide as disclosed herein.

Implantable devices of the present disclosure are useful in the treatment of a disease. In some embodiments, the pH of the formulation is at a level optimizing the stability of the therapeutic agent. In some embodiments, the formulation is at a level optimizing the rate of release of the therapeutic agent through the titania nanoporous membrane.

In some embodiments, the formulation is at a level optimizing both the stability of the therapeutic agent and the rate of release of the therapeutic agent through the nanoporous membrane.

In some instances, the release rate and stability are optimized such that the treatment will last for at least 10 days, at least 30 days, at least 45 days, at least 60 days, at least 75 days, at least 90 days, or at least 120 days or even longer.

In some instances, the therapeutic agent is an incretin mimetic. In some instances, the incretin mimetic is exenatide. In some instances, the therapeutic agent treats a disease such as Type II Diabetes, Obesity, or Non-Alcoholic Steatohepatitis. In other instances, the therapeutic agent is effective to restore normoglycemia, achieve a sustained delay in the progression of, or an amelioration of diabetes in a subject, or a delay in diabetes onset in a subject at risk for diabetes.

Devices of the present disclosure are useful in methods for the treatment of a disease. Such methods may comprise providing the device described herein for the treatment of a disease, and implanting the device in a subject in need of treatment. In some embodiments, the pH of the formulation is at a level optimizing the stability of the therapeutic agent. In some embodiments, the formulation is at a level optimizing the rate of release of the therapeutic agent through the titania nanoporous membrane.

In some embodiments, the formulation is at a level optimizing both the stability of the therapeutic agent and the rate of release of the therapeutic agent through the titania nanoporous membrane.

In some instances, the release rate and stability are optimized such that the treatment will last for at least 10 days, at least 30 days, at least 45 days, at least 60 days, at least 75 days, at least 90 days, or at least 120 days or even longer.

In some instances, the therapeutic agent is an incretin mimetic. In some instances, the incretin mimetic is exenatide. In some instances, the therapeutic agent treat a disease such as Type II Diabetes, Obesity, or Non-Alcoholic Steatohepatitis.

Implantable device of the present disclosure are useful in a method for reducing the plasma level of hemoglobin A1C in a subject in need of improvement of glycemic control, the method comprising:
providing an implantable device described herein, and implanting the device in the subject, with a concomitant decrease in A1C plasma levels.

In one aspect, the decrease is measured from when the subject is placed on an implanted device compared to subcutaneous administration. The implanted device decreases the subject's HbAlc level.

In certain instances, the HbA1c levels of the subject can be from about 1% to about 12%. Typically for a normal individual, the HbA1c levels are less than about 5.6% such as about 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, or about 5.6%.

Levels of HbAlc just below 6.5% such as 5.7% 5.8, 5.9, 6, 6.1, 6.2, 6.3, 6.4, may indicate the presence of intermediate hyperglycemia.

HbAlc levels ≥6.5%, such as 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 10.1, 10.2, 10.3, 10.4, 10.5, 10.6, 10.7, 10.8, 10.9, 11, 11.1, 11.2, 11.3, 11.4, 11.5, 11.6, 11.7, 11.8, 11.9, and/or 12% indicates diabetes. In contrast to the common plasma glucose tests, the level of glycated hemoglobin is not influenced by daily fluctuations in the blood glucose concentration, but reflect the average glucose levels over the prior six to eight weeks.

In certain instances, the decrease in HbA1c level using the implanted device compared to subcutaneous injection is at least about 0.1% to about 5.0 % of the HbA1c level. In certain instances, the decrease in HbAlc is about 0.1%, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, or 5.0%.

### Examples

Using drug delivery devices and methods of manufacture as described in PCT/US2021/019559, published as WO 2021/173770, a series of experiments with formulations of exenatide at different levels of pH was performed.

Briefly: Cylindrical polycarbonate capsules with reservoirs of about 27 microliter or about 50 microliter were fitted with a pierceable silicone septum on one end and a titanium screw cap holding a titania nanotube membrane on the other end. In these laboratory experiments polycarbonate capsules were used to allow for visual inspection of their contents. In some instances, dry formulations of a therapeutic agent, exenatide, were filled into the reservoirs, in some instances together with insoluble polymeric stabilizers, after which the capsules were sealed by attaching the titanium screw cap. Appropriate solvents were filled into the devices by means of a syringe needle piercing the septum. In some instances, the therapeutic agent was dissolved in an appropriate buffer system after which the solution was filled into the reservoirs. Filling of the reservoirs was facilitated by applying reduced pressure through the membrane during the hydration process.

Membranes were prepared according to US Patent No. 9814867. All membranes received at least 20 cycles of atomic layer deposition according to US Patent No. 9770412. Membrane diameter was about 0.3 mm with about 6,000,000 nanopores per membrane; the pore diameter at the titanium substrate end was in the order of 28 nm. Membrane diameter was about 0.3 mm with about 6,000,000 nanopores per membrane; the pore diameter at the titanium substrate end was in the order of 28 nm.

The exenatide used was obtained in the form of exenatide acetate from Bachem Americas, Inc., 3132 Kashiwa Street, Torrance, CA 90505, USA

### Example 1

Polycarbonate capsules with an internal reservoir volume of about 50 microliter were fitted with a silicone septum on one end, and with a titanium screw cap holding a titania nanotube membrane on the other end. Before capping the capsules, the reservoirs were filled with approximately 10.8 mg of exenatide. In order to bring the exenatide in the devices in solution, a vacuum was applied to the reservoirs through the membrane, after which a 0.5M citrate buffer at pH 3.1 was injected into the reservoirs by piercing the septum with a syringe needle attached to a pump system delivering the citrate buffer. Previous studies had shown that the resulting pH of the solution in the reservoir would be about 3.6.

The devices were submerged and stirred in 10 ml of release rate buffer on a USP-App7 release rate tester.

The initial release rate buffer was 0.5 M citric acid buffer, pH 3.6. (FIG. 2) After 14 days the release rate buffer was switched to phosphate buffered saline (PBS) at pH 7.4. A significant increase in release rate was observed. At day 28, the release rate buffer was switched back to citrate pH 3.6, and a drop in release rate was observed. At day 29, the release rate buffer was again switched to PBS pH 7.4, causing an increase in release rate, and at day 30 the release rate buffer was switched again to citrate pH 3.6, and the release rate dropped.

### Example 2

Polycarbonate capsules with an internal reservoir volume of about 27 microliter were fitted with a silicone septum on one end, and with a titanium screw cap holding a titania nanotube membrane on the other end. Before capping the capsules, the reservoirs were filled with approximately 5.7 mg of exenatide. In order to bring the exenatide in the devices in solution, a vacuum was applied to the reservoirs through the membrane, after which citrate buffers were injected into the reservoirs by piercing the septum with a syringe needle attached to a pump system delivering the buffers. The citrate buffers were either a 0.5M buffer at pH 3.6 containing 0.02M Tween 20, or a 0.5M buffer at pH 5.6 containing 0.02 M Tween 20.

The devices were submerged and stirred in 10 ml of release rate buffer on a USP-App7 release rate tester. The release rate media were either phosphate buffered saline or carbonate buffered saline at pH 7.4.

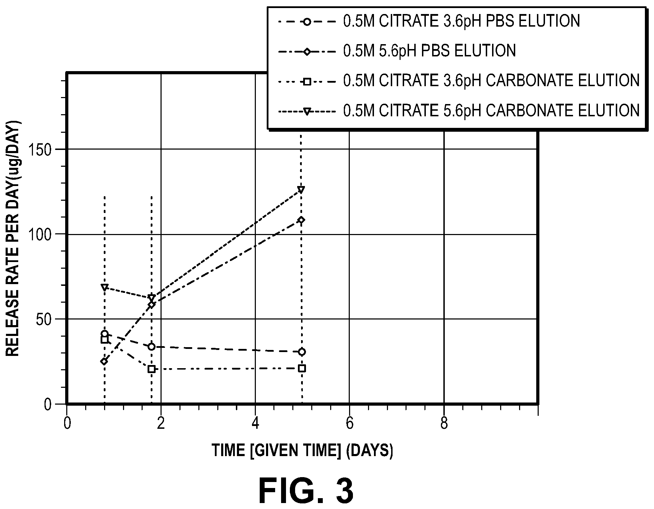

As can be seen in FIG. 3, the release from the devices filled with the pH of 3.6 buffer was substantially slower than the release from devices filled with the pH 5.6 buffer. The effect was similar for the phosphate buffered release rate medium and for the carbonate buffered release rate medium.

### Example 3

Polycarbonate capsules with an internal reservoir volume of about 52 microliter were fitted with a silicone septum on one end, and with a titanium screw cap holding a titania nanotube membrane on the other end. In this experiment the devices were filled with pre-made exenatide solutions. The exenatide solutions contained 25% exenatide by weight, and were adjusted to target pH levels of 4.5, 5.0. 5.5 and 6.0. solutions were prepared in 0.9% NaCl and were pH adjusted with NaOH or HCl. In order to fill the devices with the exenatide solutions, a vacuum was applied to the reservoirs through the membrane, after which the exenatide solutions were injected into the reservoirs by piercing the septum with a syringe needle attached to a pump system delivering the citrate buffer

The devices were submerged and stirred in 10 ml of release rate buffer on a USP-App7 release rate tester. The results are shown in FIG. 4. After a brief initial burst of release, possibly caused by the presence of residual exenatide on the outside of the device, such as in the threads of the threaded cap, a clear dependence of the release rate on the formulation was observed. At pH 4.5 the release rate remained in a range of about 1 - 5 mcg per day, while at pH 6 the rates rose to between 60 and 80 mcg per day, so a factor of at least 10X higher.

### Example 4

Polycarbonate capsules with an internal reservoir volume of about 50 microliter were fitted with a silicone septum on one end, and with a titanium screw cap holding a titania nanotube membrane on the other end. Before capping the capsules, the reservoirs were filled with approximately 10 mg of exenatide and 10mg of a pH stabilizing ion exchange resin, Diaion WK40L (See U.S. Provisional Patent Application No. 62/983,296). In order to bring the exenatide in the devices in solution, a vacuum was applied to the reservoirs through the membrane, after which a citrate buffer (0.2M, pH 5.2, 0.27% Tween in Water For Injection) was injected into the reservoirs by piercing the septum with a syringe needle attached to a pump system delivering the buffer. The final pH was recorded at 4.9.

In-vitro release rate experiments were performed in 4 mL HPLC vials containing 3 mL of Bis-Tris buffer at pH 7.4. The vials were shaken in a shaker plate in an incubator at 37C. At the end of the time period devices were terminated, disassembled and the internal pH measured.

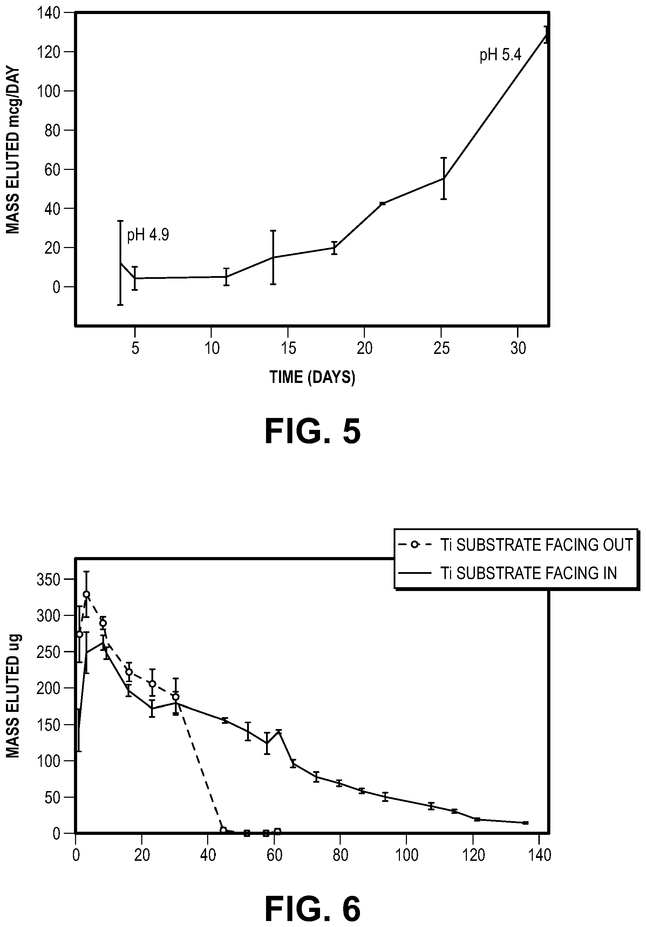

As can be seen in FIG. 5, the initial release rates were about 5b mcg/day. At 28 days the internal pH had risen to 5.4, and the release rate had increased to about 120 mcg/day.

### Example 5

Polycarbonate capsules with an internal reservoir volume of about 50 microliter were fitted with a silicone septum on one end, and with a titanium screw cap holding a titania nanotube membrane on the other end. Before capping the capsules, the reservoirs were filled with approximately 15 mg of exenatide. No additional pH stabilizer was used in this study. In order to bring the exenatide in the devices in solution, a vacuum was applied to the reservoirs through the membrane, after which a citrate buffer (0.5M, 0.27% Tween in Water For Injection, pH = 5.05) was injected into the reservoirs by piercing the septum with a syringe needle attached to a pump system delivering the buffer.

One group of devices had the membranes attached with the titanium substrate facing in towards the reservoir, the other group had the membranes attached with the titanium substrate facing out, towards the release rate medium.

The devices were submerged and stirred in 10 ml of release rate buffer (Phosphate buffered saline at pH 7.4) on a USP-App7 release rate tester.

As can be seen in FIG. 6, the group with the membranes mounted with the titanium substrate facing out showed a rapid decline in release rate around day 30. The other group had a much slower, gradual decline over 135 days.

Embodiments of the disclosure therefore provide powerful methods to control the release of therapeutic agents from implantable devices by determining the dependence of the rate of release of the agents on the pH of the formulation inside the devices, and then preparing those formulations at a pH that will provide the desired release rate.

### Example 6

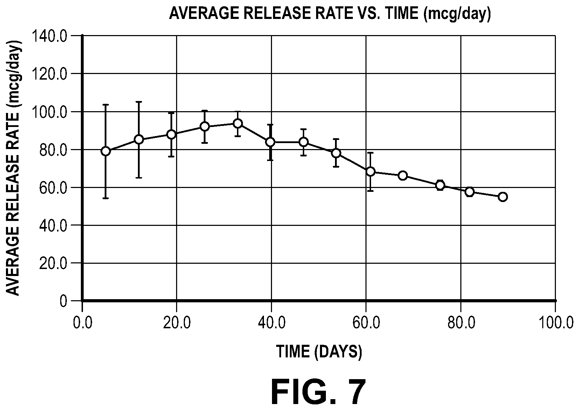

In results of this example are shown in FIG. 7. A titanium reservoir is used instead of a polycarbonate reservoir. The titanium reservoir had a length of approximately 25 mm and a diameter of approximately 2.25 mm. A titanium substrate with a titanium oxide nanoporous membrane was welded to one end of the device. The nanoporous membrane had a diameter of 0.3 mm and was composed of about 6,000,000 nanopores. The average diameter of the nanopores at the substrate end was approximately 20 nm.

A silicone septum was inserted at the other end of the reservoir. The reservoir contained about 10 mg of cross-linked polymethacrylate beads (Purolite C115, Purolite, Inc, 2201 Renaissance Boulevard, King of Prussia, PA 19406, USA) with a pH adjusted to 5.5 by titration with NaOH. About 46 mg of a formulation containing 24% exenatide-acetate (w/w), 0.25% Polysorbate 20, 154 mM Na⁺ and a pH of 5.5 was filled into the device as per methods in PCT/US2021/019559, published as WO 2021/173770.

Release rate testing was performed by submerging the devices in 3 ml of a 26 mM bis-tris buffer, 154 mM NaCl on a shaker plate at 37°C and measuring the amounts released at regular intervals by reverse phase HPLC. The resulting release rate profile (n=8) is shown in FIG. 7. The error bars indicate standard deviations.

It is to be understood that the above description is intended to be illustrative and not restrictive. Many embodiments will be apparent to those of skill in the art upon reading the above description.

## Claims

1. Use of a pH controlling agent to control a rate of release of a therapeutic agent, which is a peptide or protein, from an implantable device, the device comprising:
a capsule configured for implantation and having a reservoir;
a fluid formulation of the therapeutic agent contained within the reservoir and having a pH; and
a titania nanoporous membrane with a plurality of pores attached to the capsule and providing a diffusion path for release of the therapeutic agent out of the reservoir;
wherein the rate of release of the therapeutic agent through the nanoporous membrane is dependent on the pH of the formulation; and
wherein the pH of the formulation is selected at a level suitable to achieve a desired rate of release of the therapeutic agent.

2. Use of claim 1, wherein the peptide or protein is an incretin mimetic.

3. Use of claim 2, wherein the incretin mimetic is selected from exenatide, liraglutide, semaglutide, cotadutide, dulaglutide, albiglutide, lixisenatide, sitagliptin, saxagliptin, alogliptin, or linagliptin.

4. Use of claim 2 or 3, wherein the incretin mimetic is exenatide.

5. Use of any one of claims 1-4, wherein the therapeutic agent is exenatide, and wherein the pH is between 4.0 and 6.5.

6. Use of any one of claims 1-5, wherein the therapeutic agent is exenatide, and wherein the pH is between 4.5 and 6.0.

7. Use of any one of claims 1-6, wherein the pH controlling agent is an insoluble polymeric stabilizer.

8. Use of claim 7, wherein the insoluble polymeric stabilizer contains monomeric units of acrylic acid, methacrylic acids, or both.

9. Use of any one of claims 1 to 8, wherein the fluid formulation of the therapeutic agent further comprises the pH controlling agent.

10. Use of any one of claims 1 to 6, wherein the pH controlling agent is a low molecular weight pH stabilizer or a high molecular weight compound.

11. Use of claim 10, wherein:
the pH controlling agent is a low molecular weight pH stabilizer selected from: (a) an acidic compound, which is optionally a weakly acidic compound; and (b) a basic compound, which is optionally a weakly basic compound; or
the pH controlling agent is a high molecular weight compound selected from a poly-acid or a poly-base.

12. Use of any one of claims 1 to 6, wherein the pH controlling agent is a buffer selected from the group consisting of citrate/citric acid, acetate/acetic acid, phosphate/phosphoric acid, formate/formic acid, propionate/propionic acid, lactate/lactic acid, carbonate/carbonic acid, ammonium/ammonia, edentate/edetic acid, and combinations thereof.

13. Use of any one of claims 1 to 12, wherein the therapeutic agent is an agent for treating a disease selected from Type II diabetes, obesity, and non-alcoholic steatohepatitis.

## Patentansprüche

1. Verwendung eines Mittels zum Steuern des pH-Wertes für die Steuerung der Freisetzungsrate eines therapeutischen Mittels, das ein Peptid oder Protein ist, aus einer implantierbaren Vorrichtung, wobei die Vorrichtung umfasst:
eine Kapsel, die für die Implantation konfiguriert ist und ein Reservoir aufweist;
eine Fluidformulierung des therapeutischen Mittels, die in dem Reservoir enthalten ist und einen pH-Wert aufweist; und
eine nanoporöse Titandioxidmembran mit einer Mehrzahl von Poren, die an der Kapsel angebracht ist und einen Diffusionsweg für die Freisetzung des therapeutischen Mittels aus dem Reservoir bereitstellt;
wobei die Freisetzungsrate des therapeutischen Mittels durch die nanoporöse Membran von dem pH-Wert der Formulierung abhängt; und
wobei der pH-Wert der Formulierung auf einem Niveau ausgewählt wird, das geeignet ist, um eine gewünschte Freisetzungsrate des therapeutischen Mittels zu erreichen.

2. Verwendung nach Anspruch 1, wobei das Peptid oder Protein ein Inkretinmimetikum ist.

3. Verwendung nach Anspruch 2, wobei das Inkretinmimetikum aus Exenatid, Liraglutid, Semaglutid, Cotadutid, Dulaglutid, Albiglutid, Lixisenatid, Sitagliptin, Saxagliptin, Alogliptin oder Linagliptin ausgewählt ist.

4. Verwendung nach Anspruch 2 oder 3, wobei das Inkretinmimetikum Exenatid ist.

5. Verwendung nach einem der Ansprüche 1-4, wobei das therapeutische Mittel Exenatid ist und wobei der pH-Wert zwischen 4,0 und 6,5 liegt.

6. Verwendung nach einem der Ansprüche 1-5, wobei das therapeutische Mittel Exenatid ist und wobei der pH-Wert zwischen 4,5 und 6,0 liegt.

7. Verwendung nach einem der Ansprüche 1-6, wobei das Mittel zum Steuern des pH-Wertes ein unlöslicher polymerer Stabilisator ist.

8. Verwendung nach Anspruch 7, wobei der unlösliche polymere Stabilisator Monomereinheiten von Acrylsäure, Methacrylsäuren oder beiden enthält.

9. Verwendung nach einem der Ansprüche 1 bis 8, wobei die Fluidformulierung des therapeutischen Mittels ferner das Mittel zum Steuern des pH-Wertes umfasst.

10. Verwendung nach einem der Ansprüche 1 bis 6, wobei das Mittel zum Steuern des pH-Wertes ein pH-Wertstabilisator niedrigem Molekulargewicht oder eine Verbindung mit hohem Molekulargewicht ist.

11. Verwendung nach Anspruch 10, wobei:
das Mittel zum Steuern des pH-Wertes ein pH-Wertstabilisator mit niedrigem Molekulargewicht ist, ausgewählt aus: (a) einer sauren Verbindung, die optional eine schwach saure Verbindung ist; und (b) einer basischen Verbindung, die optional eine schwach basische Verbindung ist; oder
das Mittel zum Steuern des pH-Wertes eine Verbindung mit hohem Molekulargewicht ist, ausgewählt aus einer Polysäure oder einer Polybas.

12. Verwendung nach einem der Ansprüche 1-6, wobei das Mittel zum Steuern des pH-Wertes ein Puffer ist, der aus der Gruppe ausgewählt ist, bestehend aus Citrat/Zitronensäure, Acetat/Essigsäure, Phosphat/Phosphorsäure, Formiat/Ameisensäure, Propionat/Propionsäure, Lactat/Milchsäure, Carbonat/Kohlensäure, Ammonium/Ammoniak, Edetat/Edetinsäure und Kombinationen davon.

13. Verwendung nach einem der Ansprüche 1-12, wobei das therapeutische Mittel ein Mittel zum Behandeln einer Krankheit ist, ausgewählt aus Typ-II-Diabetes, Adipositas und nichtalkoholischer Steatohepatitis.

## Revendications

1. Utilisation d'un agent régulateur de pH pour réguler la vitesse de libération d'un agent thérapeutique, consistant en un peptide ou une protéine, à partir d'un dispositif implantable, le dispositif comprenant :
une capsule conçue pour être implantée et dotée d'un réservoir,
une formulation fluide dudit agent thérapeutique contenue au sein du réservoir et présentant un pH, et
une membrane nanoporeuse en dioxyde de titane présentant une pluralité de pores et fixée à la capsule, fournissant un trajet de diffusion pour la libération de l'agent thérapeutique hors du réservoir ;
la vitesse de libération de l'agent thérapeutique à travers la membrane nanoporeuse dépendant du pH de la formulation, et
le pH de la formulation étant sélectionné à un niveau approprié pour obtenir une vitesse de libération souhaitée de l'agent thérapeutique.

2. Utilisation selon la revendication 1, le peptide ou la protéine étant un mimétique de l'incrétine.

3. Utilisation selon la revendication 2, le mimétique de l'incrétine étant sélectionné parmi : exénatide, liraglutide, semaglutide, cotadutide, dulaglutide, albiglutide, lixisénatide, sitagliptine, saxagliptine, alogliptine ou linagliptine.

4. Utilisation selon la revendication 2 ou 3, le mimétique de l'incrétine étant l'exénatide.

5. Utilisation selon l'une quelconque des revendications 1 à 4, l'agent thérapeutique étant l'exénatide et le pH étant situé entre 4,0 et 6,5.

6. Utilisation selon l'une quelconque des revendications 1 à 5, l'agent thérapeutique étant l'exénatide et le pH étant situé entre 4,5 et 6,0.

7. Utilisation selon l'une quelconque des revendications 1 à 6, l'agent régulateur de pH étant un stabilisateur polymère insoluble.

8. Utilisation selon la revendication 7, le stabilisateur polymère insoluble contenant des unités monomères d'acide acrylique, d'acides méthacryliques, ou les deux.

9. Utilisation selon l'une quelconque des revendications 1 à 8, la formulation fluide dudit agent thérapeutique comprenant en outre l'agent régulateur de pH.

10. Utilisation selon l'une quelconque des revendications 1 à 6, l'agent régulateur de pH étant un stabilisateur de pH de faible poids moléculaire ou un composé de poids moléculaire élevé.

11. Utilisation selon la revendication 10, dans le cadre de laquelle :
l'agent régulateur de pH est un stabilisateur de pH de faible poids moléculaire sélectionné parmi : (a) un composé acide consistant éventuellement en un composé faiblement acide, et (b) un composé basique consistant éventuellement en un composé faiblement basique, ou
l'agent régulateur de pH est un composé de poids moléculaire élevé sélectionné parmi un polyacide ou une polybase.

12. Utilisation selon l'une quelconque des revendications 1 à 6, l'agent régulateur de pH étant un tampon sélectionné dans le groupe constitué par : citrate/acide citrique, acétate/acide acétique, phosphate/acide phosphorique, formiate/acide formique, propionate/acide propionique, lactate/acide lactique, carbonate/acide carbonique, ammonium/ammoniaque, édétate/acide édétique et des combinaisons de ceux-ci.

13. Utilisation selon l'une quelconque des revendications 1 à 12, l'agent thérapeutique étant un agent destiné au traitement d'une maladie sélectionnée parmi le diabète de type II, l'obésité et la stéatohépatite non alcoolique.
